# EUROPÄISCHE PATENTANMELDUNG

(11) **EP 1 408 441 A2**
(43) Veröffentlichungstag der Anmeldung: **14.04.2004**
(21) Anmeldenummer: 03020130.5
(22) Anmeldetag: 05.09.2003
(51) Int. Cl.: G06K 9/20, A61B 5/117

(54) **Vorrichtung zur berührungslosen optischen Erfassung biometrischer Eigenschaften wenigstens eines Körperteils**

(30) Priorität: 05.10.2002 DE 10246411
(71) Anmelder: ASTRA Gesellschaft für Asset Management mbH & Co. KG, 30890 Barsinghausen (DE)
(72) Erfinder: Stobbe, Anatoli, D-30890 Barsinghausen, Bundesrepublik (DE)
(74) Vertreter: Patentanwälte Thömen & Körner

(57) **Zusammenfassung**

Es wird eine Vorrichtung zur berührungslosen optischen Erfassung biometrischer Eigenschaften wenigstens eines Körperteils mit wenigstens einer ein Linsensystem und einen Bildsensor umfassenden Kamera durch Aufnahme eines Oberflächenbildes des in einer Gegenstandsebene positionierbaren Körperteils beschrieben.

Das Linsensystem bildet im gesamten Bildfeld verzeichnungsfrei ab und besitzt eine Schärfentiefe, die wenigstens der maximalen topografischen Ausdehnung der Oberflächenkontur der von der Kamera aus einer Richtung erfassbaren Oberfläche des Körperteils entspricht. Die Vorrichtung umfasst eine Positionierhilfe, mittels der Anweisungen an einen Benutzer erzeugbar sind, um die abzubildende Oberfläche des Körperteils in der Gegenstandsebene berührungslos zu positionieren.

## Beschreibung

Die Erfindung betrifft eine Vorrichtung zur berührungslosen optischen Erfassung biometrischer Eigenschaften wenigstens eines Körperteils nach dem Oberbegriff des Anspruchs 1.

Um eine Zugangsberechtigung von Personen zu gesicherten Objekten, wie Gebäuden, Räumen, Wertschränken, Geldautomaten oder Computern nachzuweisen, können neben Berechtigungskartenmit automatisch lesbaren Codes sowie der manuellen Eingabe von Codes auch biometrische Daten der berechtigten Personen ausgewertet werden. Biometrische Daten bieten gegenüber den anderen Nachweisarten den Vorteil, dass sie an die berechtigte Person individuell gebunden sind und nicht durch Entwendung, wie bei Berechtigungskarten oder durch Ausforschen, wie bei manuell einzugebenden Codes, von Dritten übernommen werden können.

Aus der EP 1 073 988 B1 ist ein System zur berührungslosen Hand- und Fingerlinienerkennung bekannt. Mittels einer Kamera werden die Hand- und Fingerlinien einer Person optisch erfasst und elektronisch ausgewertet. Um sicher zu stellen, dass sich die von der Kamera aufzunehmende Fläche in der Gegenstandebene der Kamera befindet, damit sie auf der Bildebene der Kamera scharf abgebildet wird sind mehrere Lösungsvarianten angegeben.

Bei einer Ausführung muss die Hand oder der Finger auf eine Stütze aufgelegt werden. Gemäß einer anderen Ausführung ist eine Schablone mit einer ausgesparten Handkontur vorgegeben, in die eine Hand hineingehalten werden muss. Eine weitere Ausführung sieht die Projektion von zwei Lichtbildern auf die Hand vor, die durch Veränderung der Position der Hand zur Deckung zu bringen sind. Schließlich wird noch vorgeschlagen, mittels Hologramm ein Luftbild einer Hand zu erzeugen, das die Position für die Hand einer zu identifizierenden Person vorgibt. Die Lösungen für eine berührungslose Positionierung beziehen sich auf die Hand als Ganzes aber nicht gesondert auf die abzubildende Handfläche.

Der Erfindung liegt die Aufgabe zugrunde, eine Vorrichtung der eingangs genannten Art zu schaffen, die eine verbesserte Auflösung und berührungslose optische Erfassung von biometrischen Eigenschaften wenigstens eines Körperteils mittels der Kamera ermöglicht und dadurch die Voraussetzungen für eine bessere und sicherere Auswertung und Unterscheidung schafft.

Diese Aufgabe wird bei einer Vorrichtung nach dem Oberbegriff des Anspruchs 1 durch die Merkmale des Anspruchs 1 gelöst.

Weiterbildungen und vorteilhafte Ausgestaltungen ergeben sich aus den Unteransprüchen.

Durch die Nutzung eines Linsensystems, das im gesamten Bildfeld verzerrungsfrei abbildet, ergibt sich eine maßstabsgetreue Wiedergabe der biometrischen Eigenschaften des Körperteils auf der Bildebene der Kamera, so dass unabhängig von der Lage des Körperteils in der Gegenstandsebene die Merkmale gleich abgebildet werden. Der Abbildungsmaßstab ist also am Bildrand genau so wie in der Bildmitte. Auf eine Entzerrung, wie sie z. B. bei einem Weitwinkel- oder Fischaugen-Linsensystem nötig wäre, kann somit verzichtet werden. Die bei der Auswertung anzuwendenden Algorithmen lassen sich so einfacher gestalten, was die für die Auswertung benötigte Rechenzeit verringert und damit auch die Verzögerungszeit zwischen dem Beginn der Aufnahme der biometrischen Eigenschaften und dem Ergebnis der Auswertung.

Das weitere Merkmal, dass die Schärfentiefe wenigstens der maximalen topographischen Oberflächenkontur der von der Kamera aus einer Richtung erfassten Oberfläche des Körperteils entspricht, ermöglicht eine scharfe flächenmäßige Abbildung des Körperteils trotz seiner dreidimensionalen Ausdehnung. Dadurch werden nicht nur die biometrischen Eigenschaften des Bereichs des Körperteils scharf abgebildet, die sich exakt in der Gegenstandsebene des Linsensystems befinden, sondern auch diejenigen Bereiche, die gegenüber der exakten Gegenstandsebene dichter am Linsensystem oder weiter weg liegen. Auch unvermeidliche Toleranzen bei der freien, nicht unterstützten Positionierung des Körperteils im Raum führen zu keiner negativen Verminderung der Schärfe der Abbildung.

Durch die Positionierhilfe wird dem Anwender eine Anweisung übermittelt, die abzubildende Oberfläche des Körperteils in der Gegenstandsebene zu positionieren. Im Gegensatz zur im Stand der Technik diskutierten Lösung wird damit nicht das Körperteil nur als Ganzes, also unabhängig von seiner dreidimensionalen Ausdehnung ausgerichtet, sondern die abzubildende Fläche wird möglichst exakt in der Gegenstandsebene positioniert.

Dies hat den Vorteil, dass die Schärfentiefe des Linsensystems der Kamera gerade so groß bemessen werden muss, dass nur unvermeidliche Toleranzen bei der frei im Raum vorzunehmenden Positionierung sowie das maximale topographische Oberflächenprofil berücksichtigt werden müssen. Es lassen sich dadurch größeren Blendenöffnungen oder geringere Belichtungszeiten realisieren, als bei einer Auslegung der Schärfentiefe des Linsensystems, die alle denkbaren Abweichungen von der Gegenstandsebene berücksichtigen würde.

Gemäß einer Weiterbildung umfasst die Schärfentiefe des Linsensystems zusätzlich einen Bereich, der Ungenauigkeiten der Position der abzubildenden Oberfläche des Körperteils durch Abweichungen des Abstandes, durch Drehung oder durch Neigung gegenüber einer Idealposition entspricht.

Es werden dadurch Toleranzen berücksichtigt, die durch eine individuelle Haltung des Körperteils verursacht werden und sich nicht oder nur schwer durch Anweisungen an den Benutzer beseitigen lassen.

Vorzugsweise ist das Verhältnis aus Gegenstandsweite zu Brennweite größer 10 bemessen.

Mit dieser Bemessungsregel lässt sich bei vorgegebener Brennweite eine Gegenstandsweite angeben, bei der eine ausreichende Schärfentiefe unter Berücksichtigung des vorgenannten Toleranzbereichs erzielt wird.

Im Strahlengang zwischen der Gegenstandsebene und dem Linsensystem können mehrere Umlenkspiegel angeordnet sein.

Dies ermöglicht auch bei großen Gegenstandsweiten einen kompakten Aufbau.

Die biometrischen Eigenschaften des Körperteils oder der Körperteile können von einer der Seiten oder mehreren Seiten erfassbar sein.

Es lassen sich auf diese Weise größere Bereiche oder zusätzliche räumliche Merkmale erfassen und damit die Erkennungssicherheit steigern. Außerdem können so auch mehrere Körperteile in einem Vorgang erfasst werden. Geeignete Körperteile sind Hände oder Finger. Diese können von innen, also mit Handlinien oder Fingerlinien, von oben, von vorn oder von der Seite erfasst werden.

Die Erfassung der Körperteile von mehreren Seiten ist durch wenigstens eine Kamera durchführbar und die Auswertung der Aufnahmen erfolgt einzeln oder in Kombination.

Es lassen sich so die biometrischen Merkmale aus unterschiedlichen Blickwinkeln einzeln auswerten oder durch Überlagerung eine Gesamtauswertung durchführen.

Die Positionierhilfe kann eine Lichtquelle umfassen, welche einen gebündelten Lichtstrahl im sichtbaren Bereich für eine Mittenfindung aussendet.

Dadurch wird dem Benutzer erleichtert, den Körperteil in der Gegenstandsebene so zu positionieren, dass eine maximale Abdeckung und Erfassung aller relevanten biometrischen Merkmale ermöglicht wird.

Weiterhin kann die Positionierhilfe einen Sensor zur Ermittlung einer Ist-Gegenstandsebene des Körperteils und eine Ausgabevorrichtung für Anweisungen zur Findung einer Soll-Gegenstandsebene umfassen.

Durch diese Positionierhilfe wird dem Benutzer ermöglicht, nach Anweisung die Positionierung des Körperteils so zu verändern, dass die abzubildende Oberfläche möglichst genau in die Gegenstandsebene zu liegen kommt und dadurch eine optimale Abbildung der biometrischen Eigenschaften auf der Bildebene der Kamera und eine entsprechend genaue Auswertung ermöglicht wird.

Bei einer vorteilhaften Ausgestaltung der Vorrichtung ist der Sensor ein Lichtschrankenarray.

Hierdurch lassen sich mit einfachen Mitteln in Abstufungen die Lage der Gegenstandsebene des Körperteils unabhängig von seiner räumlichen Ausdehnung erfassen und Signale zur Korrektur generieren.

Vorzugsweise sendet das Lichtschrankenarray Licht im nicht sichtbaren Bereich aus.

Dadurch wird der Benutzer neben der Lichtquelle mit dem gebündelten Lichtstrahl für eine Mittenfindung nicht durch zusätzliche Lichtquellen irritiert.

Alternativ kann der Sensor auch ein Abstandssensor auf kapazitiver, hochfrequenter oder Ultraschall-Basis sein.

Die Ausgabevorrichtung ist vorzugsweise eine optische oder akustische Anzeigevorrichtung.

Dadurch lassen sich Korrekturanweisungen allgemein verständlich darstellen, und unmittelbar auf entsprechende Korrekturbewegungen des Benutzers reagieren.

Gemäß einer Weiterbildung sind mittels der Kamera mehrere Aufnahmen desselben Oberflächenbildes erfassbar.

Es sind dann bei unterschiedlicher Aufnahmequalität die beste Aufnahme oder die besten Aufnahmen aus einer Aufnahmeserie auswertbar. Im Falle einzelner Aufnahmen ungenügender Qualität muss der Benutzer dann nicht die Erfassung wiederholen.

Weiterhin können mittels der Kamera mehrere Aufnahmen desselben Oberflächenbildes bei unterschiedlichen Belichtungen erfassbar und gemeinsam auswertbar sein.

Durch diese Maßnahme werden Einschränkungen der Kontrastauflösung der Kamera überwunden, indem durch unterschiedliche Belichtungen in der Gesamtheit in jedem Bildbereich ein Kontrast erzielt wird, der eine Auswertung der biometrischen Merkmale ermöglicht.

Die Vorrichtung kann eine Lichtquelle zur Beleuchtung der Oberfläche des zu erfassenden Körperteils umfassen, die aus einer Leuchtdiodenanordnung oder aus wenigstens einer Blitzlichtröhre besteht.

Hiermit lässt sich bei geringer Baugröße eine gleichmäßige Ausleuchtung des zu erfassenden Körperteils bewirken, so dass ein optimaler Kontrast erreicht wird.

Weiterhin kann die Belichtung über die Brenndauer der Leuchtdiodenanordnung oder der Blitzlichtröhre steuerbar oder regelbar sein.

Dadurch lässt sich die Belichtung auch an unterschiedliches Reflektions- und Absorptionsverhalten der zu erfassenden Körperteile anpassen, wie sie z. B. durch unterschiedliche Bräunung der Haut oder rassenspezifische Merkmale entstehen können.

Vorzugsweise sind die Lichtquellen der Positionierhilfe während der Belichtung abschaltbar.

Auf diese Weise wird eine Verfälschung der Oberfläche der zu erfassenden Körperteile durch die anderen Lichtquellen während der Oberflächenaufnahme vermieden.

Die Lichtquelle zur Beleuchtung der Oberflächen der zu erfassenden Körperteile kann weißes Licht oder monochromes Licht einer oder mehrerer wählbarer Lichtwellenlängen im sichtbaren und/oder unsichtbaren Bereich aussenden. Bei unterschiedlichen Lichtwellenlängen der Lichtquelle können mehrere Aufnahmen desselben Oberflächenbildes erfassbar sein.

Durch Anwendung alternativ von weißem Licht und von monochromem Licht einer oder mehrerer wählbarer Lichtwellenlängen lassen sich biometrische Merkmale wirksamer erkennen, die bei bestimmten Lichtwellenlängen deutlicher ausgeprägt sind. Auch lässt sich eine Optimierung bei unterschiedlichen Hauttypen der Körperteile oder rassespezifischer Unterschiede vornehmen.

Die Vorrichtung kann eine Auswertevorrichtung für die erfassten Oberflächenbilder der Körperteile umfassen, mittels der biometrische Merkmale mathematisch durch Anwendung von Rechenalgorithmen bestimmt werden. Es lassen sich auf unterschiedliche Oberflächenbilder desselben Körperteils dieselben Rechenalgorithmen anwenden oder auf dieselben Oberflächenbilder unterschiedliche Rechenalgorithmen anwenden.

Auch hierdurch wird die Auswertungsgenauigkeit verbessert, und es lässt sich eine individuelle Anpassung der Auswertemöglichkeiten für biometrische Merkmale von Personen vornehmen, die bei Menschen unterschiedlicher Hautfarbe und Rasse eine unterschiedliche Ausprägung aufweisen können.

Ferner können bei einer erstmaligen Erfassung der Oberflächenbilder des Körperteils und Anwendung von Rechenalgorithmen diejenigen Rechenalgorithmen in einem Speicher speicherbar sein, welche die besten Ergebnisse liefern. Bei einer nachfolgenden Erfassung der Oberflächenbilder sind die gespeicherten Rechenalgorithmen vorrangig anwendbar.

Hierdurch lassen sich gezielt optimale Ergebnisse erreichen und zeitaufwendige Fehlberechnungen vermeiden.

Die Vorrichtung kann zusätzliche Lichtquellen und Sensoren zur Bestimmung der Lichtdurchlässigkeit und/oder der Reflektionseigenschaften der Körperteile als zusätzliche biologische Merkmale umfassen.

Diese Merkmale können dann die biometrischen Merkmale der Oberfläche ergänzen und dadurch die Auswertung sicherer machen.

Ferner können die zusätzlichen Lichtquellen gepulstes Licht oder Licht wechselnder Intensität aussenden, und mittels der Auswertevorrichtung können die durch gepulstes Licht oder Licht wechselnder Intensität ausgelösten Signale der Sensoren ausgewertet werden.

Durch gepulstes Licht oder Licht wechselnder Intensität ergibt sich eine bessere Trennung gegen Einflüsse von Fremdlicht. Darüber hinaus können aber auch frequenzabhängige lichtdurchlässige und lichtreflektierende Eigenschaften der Körperteile als zusätzliche Merkmale erfasst werden. Schließlich ermöglicht die Anwendung von gepulstem Licht oder Licht wechselnder Intensität eine höhere Lichtamplitude in der Spitze bei vergleichsweise geringerer mittlerer Leistung, was bei Laserlicht die Gefahr einer Schädigung der Körperteile durch zu hohe Bestrahlungsleistung und der Augen durch aus der Vorrichtung tretendes Streulicht reduziert.

Gemäß einer Weiterbildung können die durch Auswertung der erfassten Oberflächenbilder der Körperteile gewonnenen biometrischen Merkmale und die zusätzlichen biometrischen Merkmale gemeinsam gespeichert werden. Durch die Ergänzung und die Vielzahl der erfassten biometrischen Merkmale wird so die Auswertungsgenauigkeit und die Zuverlässigkeit verbessert.

Weiterhin kann durch Vergleich gemessener zusätzlicher biometrischer Merkmale mit gespeicherten zusätzlichen biometrischen Merkmalen eine Manipulationserkennung durchgeführt werden.

Bei nur einer Art von zu betrachtenden biometrischen Merkmalen, z. B. der Oberfläche von Körperteilen, besteht eine Manipulationsmöglichkeit darin, die Oberflächenmerkmale einer berechtigten Person zu kopieren und auf die Oberfläche der Körperteile einer anderen nicht berechtigten Person, z. B. in Form einer Folie, zu übertragen. Weichen dann allerdings die zusätzlichen biometrischen Merkmale, wie die Lichtdurchlässigkeit oder Reflektionseigenschaften ab, so liegt nur eine unvollständige Übereinstimmung mit den gesamten biometrischen Eigenschaften vor, was auf einen Manipulationsversuch hindeuten kann.

Eine weitere Möglichkeit der Manipulationserkennung besteht darin, dass durch Vergleich gemessener biometrischer Merkmale mit gespeicherten biometrischen Merkmalen, die aufgrund von Oberflächenbildern des Körperteils bei unterschiedlichen Beleuchtungsfarben erfasst werden, auch hier unterschiedliche Eigenschaften aufgedeckt werden.

Denn wenn beispielsweise Folien mit dem grafischen Muster der biometrischen Eigenschaften einer anderen Person verwendet werden, können aufgrund der Unterschiede der optischen Eigenschaften des Fremdmaterials gegenüber den optischen Eigenschaften realer Haut Abweichungen in den Abbildungseigenschaften bei unterschiedlichen Farben auftreten, die auf diese Weise aufgedeckt werden können.

Nachfolgend wird ein Ausführungsbeispiel der Erfindung anhand der Zeichnung erläutert. In der Zeichnung zeigen:

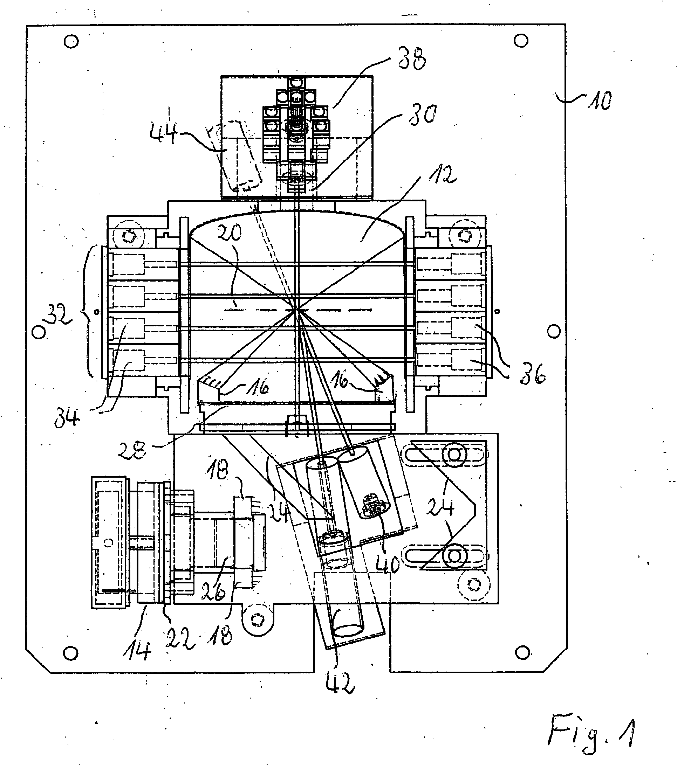

- Fig. 1: eine Frontansicht einer erfindungsgemäßen Vorrichtung und

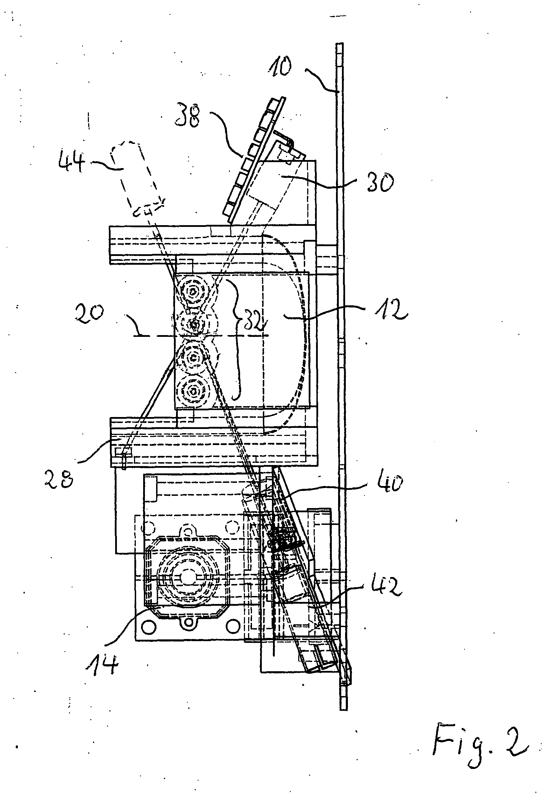

- Fig. 2: eine Seitenansicht der erfindungsgemäßen Vorrichtung.

Die Vorrichtung umfasst ein Gehäuse 10 mit einer Erfassungskammer 12 sowie einer Kamera 14 und einer Beleuchtungsvorrichtung 16, 18. In die Erfassungskammer 12 wird ein zu erfassender Körperteil, z. B. ein oder mehrere Finger oder eine Hand hineingehalten und anschließend optisch von der Kamera 14 erfasst.

Etwa waagerecht in der Mitte der Erfassungskammer 12 verläuft eine Gegenstandsebene 20 für die abzubildende Oberfläche des Körperteils. Ein in dieser Gegenstandsebene 20 befindlicher Körperteil wird auf der Bildebene der Kamera 14 abgebildet.

Der Strahlengang verläuft hier von der Gegenstandsebene 20 über Umlenkspiegel 24 zu der Kamera, die ein Linsensystem 26 und auf der Bildebene einen Bildsensor 22, z. B. einen CCD-Sensor umfasst.

Das Linsensystem 26 der Kamera 14 erzeugt ein verzeichnungsfreies Bild, d. h. alle Einzelheiten in der Gegenstandsebene 20 werden maßstabsgetreu auf der Bildebene abgebildet. Das Linsensystem 26 weist eine Brennweite mit Normalobjektiv- bis Teleobjektiveigenschaften auf, jedoch keine Weitwinkeleigenschaften, da im letzteren Falle die Verzeichnungsfreiheit nicht gewährleistet ist. Gute Abbildungseigenschaften werden bei Brennweiten von mehr als 6 Millimeter erzielt, wobei ein Brennweitenbereich zwischen 10 und 15 Millimeter vorzuziehen ist.

Die Gegenstandsweite, also der optische Abstand zwischen der Gegenstandsebene 20 und dem Linsensystem 26 beträgt wenigstens das 10 fache der Brennweite. Dadurch ergibt sich eine Schärfentiefe, die bei der 3 dimensionalen Form der Körperteile über der gesamten maximalen topographischen Oberflächenkontur der aus einer Richtung erfassbaren Oberfläche des Körperteils eine scharfe Abbildung sicherstellt.

Zur Beleuchtung der Oberfläche des zu erfassenden Körperteils dient die Beleuchtungsvorrichtung 16, 18 mit Lichtquellen in Form von Leuchtdioden. Dabei sind Leuchtdioden 16 seitlich eines Rahmens 28 im unteren Bereich der Erfassungskammer 12 angeordnet und zusätzlich Leuchtdioden 18 in unmittelbarer Nähe seitlich des Linsensystems 26 der Kamera 14. Die seitlich des Rahmens 28 angeordneten Leuchtdioden 16 bestrahlen den Körperteil mit einem großen Öffnungswinkel von der Seite her, während die in der Nähe der Kamera 14 angeordneten Leuchtdioden 18 mit einem kleinen Öffnungswinkel über die Umlenkspiegel 24 die Mitte des Körperteils beleuchten. Insgesamt wird durch diese kombinierte Anordnung der Leuchtdioden 16, 18 eine sehr gleichmäßige Ausleuchtung erreicht.

Weiterhin umfasst die Vorrichtung eine Positionierhilfe. Diese besteht aus einer Lichtquelle 30, die einen gebündelten Lichtstrahl längs einer gedachten senkrechten Ebene in der Mitte der Erfassungskammer 12 von oben nach unten richtet. Dieser Lichtstrahl trifft für den Benutzer sichtbar auf den Körperteil und erleichtert es dem Benutzer, den Körperteil so zu positionieren, dass der Lichtstrahl genau auf die Mitte fällt.

Seitlich der Erfassungskammer 12 befindet sich ein Sensor zur Ermittlung einer Ist-Gegenstandsebene des Körperteils in Form eines Lichtschrankenarrays 32. Dazu sind auf der einen Seite Leuchtdioden 34 und auf der anderen Seite Sensoren 36 angeordnet, die einen Lichtvorhang bilden. Die Sensoren 36 sind mit einer Ausgabevorrichtung in Form einer optischen Anzeigevorrichtung 38 verbunden, die Anweisungen an den Benutzer erteilt, seinen Körperteil anzuheben oder abzusenken, wenn sich die der Kamera 14 zugewandte Oberfläche nicht in der Gegenstandsebene 20 befindet.

Wird der Lichtvorhang unter der Gegenstandsebene 20 unterbrochen, so erzeugt die Anzeigevorrichtung 38 einen nach oben gerichteten Pfeil, wird der Lichtvorhang oberhalb der Gegenstandsebene 20 unterbrochen, wird ein entsprechend nach unten gerichteter Pfeil erzeugt. Die richtige Position ist erreicht, wenn der Lichtvorhang gerade im Bereich der Gegenstandsebene 20 unterbrochen wird.

Weiterhin umfasst die Vorrichtung eine zusätzliche Lichtquelle 40 und einen zusätzlichen Sensor 42 unterhalb der Erfassungskammer 12. Hiermit werden die Reflektionseigenschaften des Körperteils als zusätzliche biometrische Merkmale umfasst. Ergänzend ist ferner ein weiterer Sensor 44 vorhanden, mit dem die Lichtdurchlässigkeit erfasst werden kann. Statt einer gemeinsamen Lichtquelle kann auch eine gesonderte Lichtquelle sowie ein gesonderter Sensor verwendet werden, um Licht unterschiedlicher Wellenlängen auszuwerten. Mittels einer nicht dargestellten Auswertevorrichtung werden dann die von der Kamera gewonnen Bilder anhand von Rechenalgorithmen ausgewertet und ebenfalls die von den zusätzlichen Lichtquellen und Sensoren erfassten zusätzlichen biometrischen Eigenschaften der Lichtdurchlässigkeit und der Reflexionseigenschaften.

Um mit der Vorrichtung eine Identifizierung zu ermöglichen, werden die biometrischen Eigenschaften einer Person zunächst manipulationssicher, z. B. unter Aufsicht erfasst und auf einer Codekarte gespeichert. Bei einer späteren Identifizierung werden dann die erfassten biometrischen Eigenschaften mit der auf der Codekarte gespeicherten Eigenschaften verglichen. Dabei können auch unter einer Vielzahl möglicher biometrischer Eigenschaften einige Eigenschaften individuell festgelegt werden, die vorrangig oder mit höherer Gewichtung überprüft werden müssen. Auch können im Zuge dieser Festlegung Rechenalgorithmen bestimmt und als Information gespeichert werden, die für diese Person bessere Ergebnisse liefern als andere Rechenalgorithmen.

### Bezugszeichenliste

- 10: Gehäuse
- 12: Erfassungskammer
- 14: Kamera
- 16, 18: Beleuchtungsvorrichtung
- 20: Gegenstandsebene
- 22: Bildsensor
- 24: Umlenkspiegel
- 26: Linsensystem
- 28: Rahmen
- 30: Lichtquelle
- 32: Lichtschrankenarray
- 34: Leuchtdioden
- 36: Sensoren
- 38: Anzeigevorrichtung
- 40: Lichtquelle
- 42: Sensor
- 44: Sensor

## Patentansprüche

1. Vorrichtung zur berührungslosen optischen Erfassung biometrischer Eigenschaften wenigstens eines Körperteils mit wenigstens einer ein Linsensystem (26) und einen Bildsensor (22) umfassenden Kamera (14) durch Aufnahme eines Oberflächenbildes des in einer Gegenstandsebene (20) positionierbaren Körperteils, **dadurch gekennzeichnet, dass** das Linsensystem (26) im gesamten Bildfeld verzeichnungsfrei abbildet und eine Schärfentiefe besitzt, die wenigstens der maximalen topografischen Ausdehnung der Oberflächenkontur der von der Kamera (14) aus einer Richtung erfassbaren Oberfläche des Körperteils entspricht und die Vorrichtung eine Positionierhilfe umfasst, mittels der Anweisungen an einen Benutzer erzeugbar sind, um die abzubildende Oberfläche des Körperteils in der Gegenstandsebene (20) berührungslos zu positionieren.

2. Vorrichtung nach Anspruch 1, **dadurch gekennzeichnet, dass** die Schärfentiefe des Linsensystems (26) zusätzlich zur maximalen topografischen Ausdehnung der Oberflächenkontur der von der Kamera (14) aus einer Richtung erfassbaren Oberfläche des Körperteils einen Bereich umfasst, der Ungenauigkeiten der Position der abzubildende Oberfläche des Körperteils durch Abweichungen des Abstandes, durch Drehung oder durch Neigung gegenüber einer Idealposition entspricht.

3. Vorrichtung nach Anspruch 1 oder 2, **dadurch gekennzeichnet, dass** das Verhältnis aus Gegenstandsweite zu Brennweite größer 10 bemessen ist.

4. Vorrichtung nach einem der Ansprüche 1 bis 3, **dadurch gekennzeichnet, dass** im Strahlengang zwischen der Gegenstandsebene (20) und dem Linsensystem (26) wenigstens ein Umlenkspiegel (24) und/oder ein Umlenkprisma angeordnet sind.

5. Vorrichtung nach einem der Ansprüche 1 bis 4, **dadurch gekennzeichnet, dass** biometrischer Eigenschaften des Körperteils oder der Körperteile von einer der Seiten oder mehreren Seiten erfassbar sind.

6. Vorrichtung nach Anspruch 5, **dadurch gekennzeichnet, dass** die Erfassung der Körperteile von mehreren Seiten durch wenigstens eine Kamera (14) durchführbar ist und die Auswertung der Aufnahmen einzeln oder in Kombination erfolgt.

7. Vorrichtung nach einem der Ansprüche 1 bis 6, **dadurch gekennzeichnet, dass** die Positionierhilfe eine Lichtquelle (30) umfasst, welche einen gebündelten Lichtstrahl im sichtbaren Bereich für eine Mittenfindung aussendet.

8. Vorrichtung nach einem der Ansprüche 1 bis 7, **dadurch gekennzeichnet, dass** die Positionierhilfe einen Sensor zur Ermittlung einer Ist-Gegenstandsebene des Körperteils und eine Ausgabevorrichtung für Anweisungen zur Findung einer Soll-Gegenstandsebene umfasst.

9. Vorrichtung nach Anspruch 8, **dadurch gekennzeichnet, dass** der Sensor ein Lichtschrankenarray (32) ist.

10. Vorrichtung nach Anspruch 9, **dadurch gekennzeichnet, dass** das Lichtschrankenarray (32) Licht im nicht sichtbaren Bereich aussendet.

11. Vorrichtung nach Anspruch 8, **dadurch gekennzeichnet, dass** der Sensor ein Abstandssensor auf kapazitiver, hochfrequenter oder Ultraschall-Basis ist.

12. Vorrichtung nach einem der Ansprüche 8 bis 11, **dadurch gekennzeichnet, dass** die Ausgabevorrichtung eine optische und/oder akustische Anzeigevorrichtung (38) ist.

13. Vorrichtung nach einem der Ansprüche 1 bis 12, **dadurch gekennzeichnet, dass** mittels der Kamera (14) mehrere Aufnahmen desselben Oberflächenbildes erfassbar sind und bei unterschiedlicher Aufnahmequalität die beste Aufnahme oder die besten Aufnahmen auswertbar sind.

14. Vorrichtung nach einem der Ansprüche 1 bis 13, **dadurch gekennzeichnet, dass** mittels der Kamera (14) mehrere Aufnahmen desselben Oberflächenbildes bei unterschiedlichen Belichtungen erfassbar und gemeinsam auswertbar sind.

15. Vorrichtung nach einem der Ansprüche 1 bis 14, **dadurch gekennzeichnet, dass** die Vorrichtung eine Beleuchtungsvorrichtung (16, 18) zur Beleuchtung der Oberfläche des zu erfassenden Körperteils umfasst, die aus einer Leuchtdiodenanordnung oder aus wenigstens einer Blitzlichtröhre besteht.

16. Vorrichtung nach Anspruch 15, **dadurch gekennzeichnet, dass** die Belichtung über die Brenndauer der Leuchtdiodenanordnung oder der Blitzlichtröhre steuerbar oder regelbar ist.

17. Vorrichtung nach einem der Ansprüche 1 bis 16, **dadurch gekennzeichnet, dass** die Lichtquellen der Positionierhilfe während der Belichtung abschaltbar sind.

18. Vorrichtung nach Anspruch 15 oder 16, **dadurch gekennzeichnet, dass** die Beleuchtungsvorrichtung (16, 18) zur Beleuchtung der Oberfläche des zu erfassenden Körperteils weißes Licht oder monochromes Licht einer oder mehrerer wählbarer Lichtwellenlängen im sichtbaren und/oder unsichtbaren Bereich aussendet und dass bei unterschiedlichen Lichtwellenlängen der Lichtquelle mehrere Aufnahmen desselben Oberflächenbildes erfassbar sind.

19. Vorrichtung nach einem der Ansprüche 1 bis 18, **dadurch gekennzeichnet, dass** die Vorrichtung eine Auswertevorrichtung für die erfassten Oberflächenbilder des Körperteils umfasst, mittels der biometrische Merkmale mathematisch durch Anwendung von Rechenalgorithmen bestimmbar sind und dass auf unterschiedliche Oberflächenbilder desselben Körperteils dieselben Rechenalgorithmen anwendbar sind oder auf dieselben Oberflächenbilder unterschiedliche Rechenalgorithmen anwendbar sind.

20. Vorrichtung nach Anspruch 19, **dadurch gekennzeichnet, dass** bei einer erstmaligen Erfassung der Oberflächenbilder des Körperteils und Anwendung von Rechenalgorithmen diejenigen Rechenalgorithmen in einem Speicher speicherbar sind, welche die besten Ergebnisse liefern und bei einer nachfolgenden Erfassung der Oberflächenbilder die gespeicherten Rechenalgorithmen vorrangig anwendbar sind.

21. Vorrichtung nach einem der Ansprüche 1 bis 20, **dadurch gekennzeichnet, dass** die Vorrichtung zusätzliche Lichtquellen (40) und Sensoren (42, 44) zur Bestimmung der Lichtdurchlässigkeit und/oder der Reflexionseigenschaften des Körperteils als zusätzliche biometrische Merkmale umfasst.

22. Vorrichtung nach Anspruch 21, **dadurch gekennzeichnet, dass** die zusätzliche Lichtquellen (42, 44) gepulstes Licht oder Licht wechselnder Intensität aussenden und mittels der Auswertevorrichtung die durch gepulstes Licht oder Licht wechselnder Intensität ausgelösten Signale der Sensoren auswertbar sind.

23. Vorrichtung nach Anspruch 21 oder 22, **dadurch gekennzeichnet, dass** die durch Auswertung der erfassten Oberflächenbilder des Körperteils gewonnenen biometrischen Merkmale, und die zusätzlichen biometrischen Merkmale gemeinsam speicherbar sind.

24. Vorrichtung nach Anspruch 23, **dadurch gekennzeichnet, dass** durch Vergleich gemessener zusätzlicher biometrischer Merkmale mit gespeicherten zusätzlichen biometrischen Merkmalen eine Manipulationserkennung durchführbar ist.

25. Vorrichtung nach Anspruch 23, **dadurch gekennzeichnet, dass** durch Vergleich gemessener biometrischer Merkmale mit gespeicherten biometrischen Merkmalen, die aufgrund von Oberflächenbildern des Körperteils bei unterschiedlichen Lichtwellenlängen erfasst wurden, eine Manipulationserkennung durchführbar ist.
